# EUROPÄISCHE PATENTANMELDUNG

(11) **EP 4 458 297 A1**
(43) Veröffentlichungstag der Anmeldung: **06.11.2024**
(21) Anmeldenummer: 23171492.4
(22) Anmeldetag: 04.05.2023
(51) Int. Cl.: A61B 18/04, A61B 18/00, A61B 18/14

(54) **PLASMAINSTRUMENT MIT SEITLICHEN FENSTERN**

(71) Anmelder: Erbe Elektromedizin GmbH, 72072 Tübingen (DE)
(72) Erfinder: WALZ, Martin, 72108 Rottenburg (DE); MOSER, Sandra, 72459 Albstadt-Laufen (DE); HEYM, Johannes, 72074 Tuebingen (DE); BEUTLER, Fabian, 71654 Neckartenzlingen (DE)
(74) Vertreter: Rüger Abel Patentanwälte PartGmbB

(57) **Zusammenfassung**

Ein erfindungsgemäßes Instrument (11) zur Plasmabehandlung von biologischem Gewebe weist Plasmaaustrittsfenster in Gestalt von Öffnungen (17 - 22) mit einem zur Längsrichtung (L) schräg verlaufenden Rand auf, der durch eine Flanke (38, 39) eines Stegs gebildet sein kann. Die Stege (23 - 28) verjüngen sich in Radialrichtung (+R) und sind vorzugsweise schräg zu der Längsrichtung (L) geneigt. Die Stege (23 - 28) tragen einen proximalen Abschnitt des Instrumentenkopfs (16) und verbinden diesen nahtlos einstückig mit dem proximalen Teil des Kopfs (16). Durch die Verjüngung der Stege nach außen wird deren Abschattungswirkung minimiert oder beseitigt.

## Beschreibung

Die Erfindung betrifft ein Plasmainstrument zur Einwirkung auf biologisches Gewebe, insbesondere zur chirurgischen Behandlung von menschlichem oder tierischem Gewebe. Das Plasmainstrument ist als seitlich bzw. radial wirksames Instrument ausgebildet.

Plasmainstrumente zur Einwirkung auf biologisches Gewebe sind aus dem Stand der Technik bekannt. Beispielsweise offenbaren die DE 198 20 240 A1 wie auch die EP 1 297 082 A1 Instrumente jeweils mit einem schlauchartigen Grundkörper, durch den sich ein Lumen von seinem proximalen Ende zu seinem distalen Ende erstreckt, an dem ein Kopf mit einem oder mehreren seitlichen Fenstern angeordnet ist. Durch das Lumen verläuft ein elektrischer Leiter, der an seinem distalen Ende mit einer zentralen Elektrode verbunden ist. In Betrieb ionisiert die an eine HF-Spannungsquelle angeschlossene Elektrode durch das Lumen heranströmendes Gas, das dann als Plasmastrom seitlich austritt. In einer Ausführungsform sind zwei schlitzartige Plasmaaustrittsfenster vorgesehen, die an axial unterschiedlichen Stellen angeordnet sind und einander etwas überlappen.

Auch die EP 3 831 291 A1 offenbart ein Instrument zur Erzeugung eines Plasmas zur Einwirkung auf biologisches Gewebe. Wiederum weist das Instrument einen schlauchartigen Körper auf, der an seinem distalen Ende zumindest in einer Ausführungsform einen Kopf mit einer seitlichen Öffnung trägt, durch die ein Plasmastrom austreten kann.

Instrumente zur Erzeugung eines radial wirksamen Plasmastroms sind auch aus der EP 1 682 023 B1 sowie aus der US 9 510 889 B2 bekannt. Diese Instrumente weisen an ihrem distalen Ende eine axiale Öffnung auf, aus der eine Elektrode ragt. Die Elektrode trägt an ihrem distalen Ende einen Isolierkörper, der mit dem übrigen Instrument einen ringförmigen, sich radial öffnenden Schlitz begrenzt. Der Plasmastrom hat radial an jeder Umfangsstelle 360° freien Austritt, wobei aber der Isolierkörper von der Elektrode getragen und stabil gehalten werden muss.

Weiterer Stand der Technik kann der US 2021/259 756 A1, der EP 3 422 981 A2, der EP 3 372 183 A1, der JP 2002-2301088 A und der GB 2 573 128 A entnommen werden.

Es ist Aufgabe der Erfindung, ein robustes Plasmainstrument mit seitlich abgehendem Plasmastrom zu schaffen.

Diese Aufgabe wird mit dem Plasmainstrument nach Anspruch 1 gelöst:

Das erfindungsgemäße Plasmainstrument weist einen schlauch- oder rohrartigen Körper auf, an dessen distalen Ende ein Kopf mit wenigstens zwei Öffnungen vorgesehen ist, die mit einem sich durch den schlauchartigen Körper erstreckenden Lumen verbunden sind. Die Öffnungen sind durch einen Steg getrennt, der einen sich in einer Radialrichtung nach außen verjüngenden Querschnitt aufweist. Vorzugsweise ist in dem Kopf in der Nähe der Öffnung eine Elektrode angeordnet, die dazu eingerichtet ist, einen zur Ionisation eines Gasstroms geeigneten elektrischen Strom zu emittieren. Die Elektrode weist dabei vorzugsweise einen Durchmesser auf, der größer ist als eine in Umfangsrichtung zu messende Breite des Stegs an seiner der Elektrode zugewandten Seite. Durch diese Maßnahme und durch die Verjüngung des Stegs nach außen hin wird die Fähigkeit des Stegs minimiert, einen Plasmastrom abzuschatten bzw. zu behindern. Der Plasmastrom kann somit beide an den Steg grenzenden Öffnungen durchfließen, ungehindert von einer Öffnung zur anderen wechseln, und, wenn er durch beide Öffnungen fließt, hinter dem Steg zusammenfinden. Die Behandlung von biologischem Gewebe wird dadurch erleichtert. Ein Operateur kann das erfindungsgemäße Plasmainstrument und den davon ausgehenden Plasmastrahl ebenso leicht führen wie ein Plasmainstrument mit einem ringförmigen ununterbrochenen radialen Plasmaauslass.

Der sich nach außen verjüngende Steg kann im Querschnitt dreieckig, trapezförmig, mit geraden oder gerundeten Kanten sowie mit spitzen oder gerundeten Ecken ausgebildet sein. Vorzugsweise ist dabei die Breite des Stegs, gemessen in Umfangsrichtung, größer als die Breite der anschließenden Öffnung ebenfalls gemessen in Umfangsrichtung. Außerdem ist der Steg (sind die Stege) vorzugsweise schlank, d.h. ihre von ihrem proximalen zu ihrem distalen Ende zu messende Länge ist größer als ihr halber Umfang. Es ist aber auch möglich, an dem Kopf einen oder mehrere Stege vorzusehen, die sich nach außen nicht verjüngen. Dies kann einen, einige oder alle Stege betreffen.

Bei einem bevorzugten Instrument bilden die Öffnungen eine sich um den gesamten Kopf herum erstreckende Reihe, wobei die Öffnungen durch Stege voneinander separiert sind. Die Stege tragen das distale Ende des Kopfs, der vorzugsweise aus einem temperaturbeständigen Material, wie beispielsweise Keramik, nahtlos einstückig ausgebildet ist.

Die die Öffnungen voneinander separierenden Stege sind vorzugsweise schräg zu der Längsrichtung des Plasmainstruments ausgerichtet, d.h. in Umfangsrichtung geneigt angeordnet. Dadurch sind die Öffnungen zum Beispiel dreieckig, trapezförmig oder auch rautenförmig mit geraden oder gerundeten Kanten sowie mit geraden oder gerundeten Ecken ausgebildet. Vorzugsweise sind die Stege in Umfangsrichtung mit abwechselndem Richtungssinn geneigt, sodass die Öffnungen eine Reihe dreieckförmiger Fenster bilden, deren Spitzen bzw. schmalen Enden jeweils abwechselnd in distaler Richtung (positive Längsrichtung) oder proximaler Richtung (negative Längsrichtung) weisen.

Durch diese Maßnahme können die Öffnungen einander in Umfangsrichtung sowie in Längsrichtung überlappen. Dadurch und durch den sich radial nach außen verjüngenden Querschnitt der Stege wird eine Kontinuität des Plasmastroms erreicht, wenn dieser jeweils zu der nächstliegenden Gewebepartie fließend von einer Öffnung zur benachbarten Öffnung wechselt.

Weitere vorteilhafte Einzelheiten sind Gegenstand der Zeichnung, der zugehörigen Beschreibung oder von Ansprüchen. Es zeigen:
Figur 1 ein erfindungsgemäßes Plasmainstrument, angeschlossen an ein speisendes Gerät, in Perspektivdarstellung,
Figur 2 das distale Ende des Plasmainstruments nach Figur 1, in vergrößerter dabei aber nicht maßstäblicher Seitenansicht,
Figur 3 das Plasmainstrument nach Figur 1 und 3, geschnitten entlang der Schnittlinie III-III, in Figur 2,
Figur 4 eine abgewandelte Ausführungsform des Plasmainstruments nach Figur 2 und 3, geschnitten entsprechend der Schnittlinie III-III in Figur 2, stark vergrö-ßert,
Figur 5 eine weitere abwandelte Ausführungsform eines Plasmainstruments entsprechend Figur 1 und 2 mit abgewandelter Stegform, geschnitten entsprechend Figur 4, in vergrößerter Darstellung,
Figur 6 eine Abwicklung des aus Figur 2 ersichtlichen Kopfs zur Veranschaulichung der geometrischen Verhältnisse,
Figur 7 eine abgewandelte Ausführungsform eines erfindungsgemäßen Plasmainstrument, in geschnittener ausschnittsweiser Perspektivdarstellung,
Figur 8 eine Abwicklung des Kopfs nach Figur 7 zur Veranschaulichung der geometrischen Verhältnisse,
Figur 9 und 10 Abwicklungen weiterer alternativer Ausführungsformen erfindungsgemäßer Sonden und
Figur 11 eine Plasmasonde mit wenigstens zwei Elektroden, in ausschnittsweiser und teilweise geschnittener Darstellung ihres Kopfs.

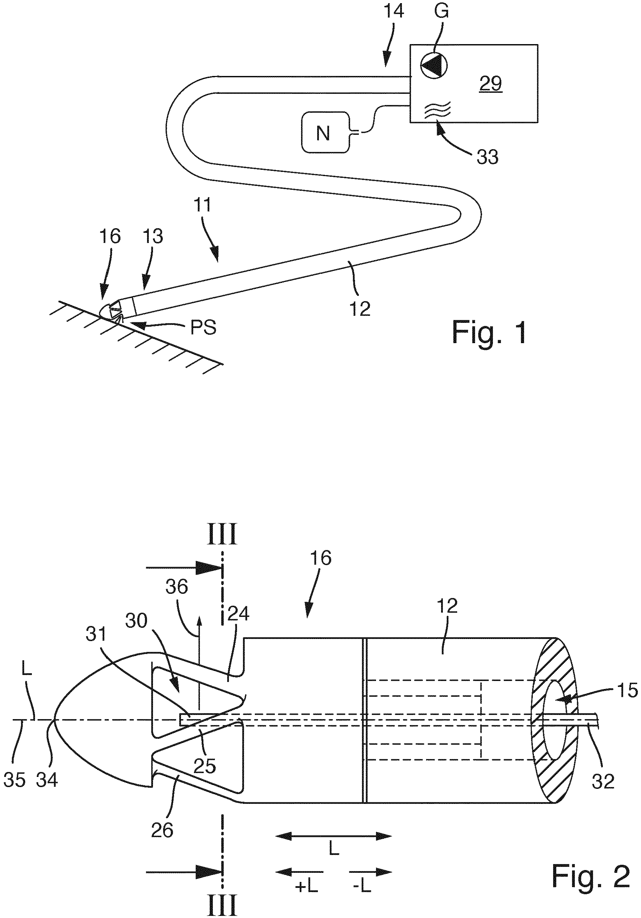

Figur 1 veranschaulicht ein als flexible Sonde ausgebildetes Plasmainstrument 11, das einen flexiblen Schlauch als Körper 12 aufweist. Der Körper 12 weist ein distales Ende 13 und ein proximales Ende 14 sowie ein Lumen 15 auf, das sich von dem proximalen Ende 14 zu dem distalen Ende 11 erstreckt. Der Körper 12 kann, wie in Figur 2 dargestellt, ein einziges Lumen 15 oder auch mehrere Lumen aufweisen. Der Körper 12 ist biegsam oder flexibel ausgebildet, um beispielsweise als Sonde durch den Arbeitskanal eines Endoskops an den Operationsort eines Patienten geschoben zu werden. Wenn das Endoskop ein steuerbares Ende aufweist, gestattet die Flexibilität des Instruments die Durchführung einer entsprechenden Abwinkelbewegung.

Das Instrument 11 kann nicht nur als endoskopisch nutzbare Sonde, sondern auch in anderer Form bereitgestellt werden, beispielsweise als laparoskopisches Instrument, wobei dann der Körper 12 ein starres Rohr ist. Auch kann das Instrument 11 für den offenchirurgischen Einsatz mit kurzem Körper 12 und am proximalen Ende vorgesehenen Handstück ausgebildet sein.

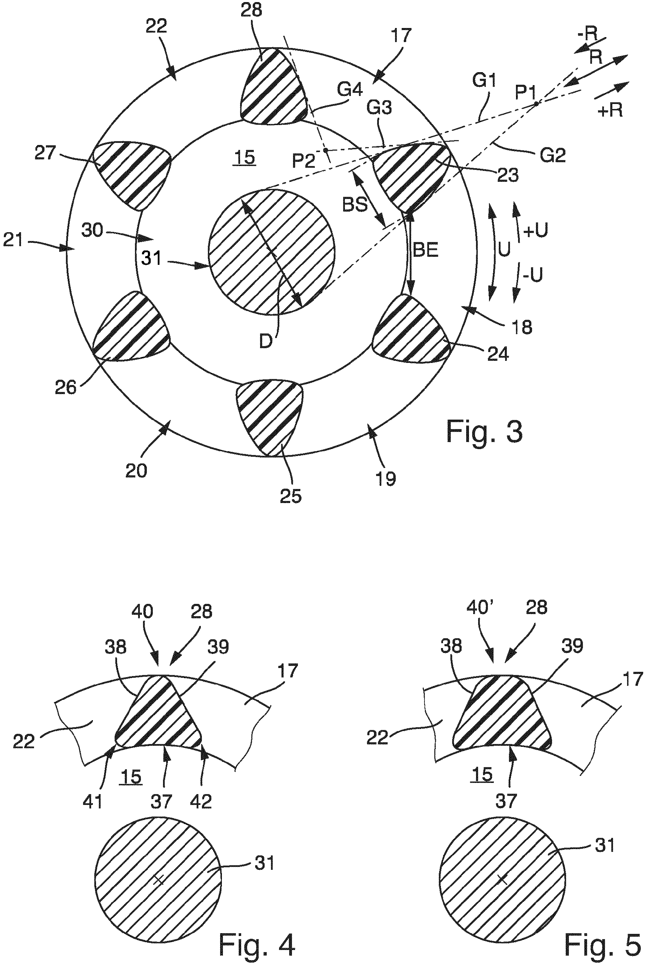

An dem distalen Ende 13 des Körpers 12 ist ein Kopf 16 angeordnet, der wenigstens zwei, vorzugsweise aber mehrere, wie aus Figur 3 hervorgeht, beispielsweise sechs Öffnungen 17 bis 22 aufweist. Alternativ können auch drei, vier, fünf, sieben, acht oder mehr Öffnungen vorgesehen sein. Die Öffnungen 17 bis 22 sind untereinander durch Stege 23 bis 28 getrennt, die, wie auch vorzugsweise der gesamte Kopf 16, aus temperaturstabilem Material, zum Beispiel Keramik, bestehen. Die Stege 23 bis 28 sind schlank, d.h. ihre Länge ist in Längsrichtung L gemessen größer, vorzugsweise mehrmals größer als ihre in Umfangsrichtung U gemessene Breite.

Das Lumen 15 ist an dem proximalen Ende 14 an ein Gerät 29 angeschlossen, das zur Versorgung und Speisung des Instruments 11 dient. Zum Beispiel kann das Gerät 19 als Gasquelle G ausgebildet sein oder eine solche enthalten. Zu diesem Zweck kann es an einen Gasspeicher, beispielsweise eine Gasflasche oder dergleichen, angeschlossen sein und Mittel zum Steuern, insbesondere zum Freigeben und Sperren, sowie gegebenenfalls zum Dosieren des Gasflusses enthalten, mit dem das Lumen 15 gespeist wird. Der Gasspeicher kann insbesondere Argon enthalten, oder auch ein anderes Inertgas, insbesondere ein zur Plasmabildung geeignetes Inertgas. Bedarfsweise kann die Sonde 29 auch zur Lieferung von aktiven Gasen, d.h. reaktiven Gasen, Aerosolen, Dämpfen oder dergleichen ausgebildet sein.

Das Lumen 15 ist mit den Öffnungen 17 bis 22 verbunden, sodass durch das Lumen zu dem Kopf 16 herangeführtes Gas aus allen Öffnungen 17 bis 22 gleichberechtigt austreten kann. Dies gilt auch, wenn mehrere Lumen in dem Körper 12 parallel zueinander vorgesehen sind.

Das Instrument 11 enthält außerdem mindestens eine Plasmaerzeugungseinrichtung 30, zum Beispiel in Gestalt einer Elektrode 31, deren distales Ende im Bereich der Öffnungen 17 bis 22 liegt. Die Elektrode kann durch einen im Wesentlichen zylindrischen Körper aus Metall gebildet sein, dessen distales Ende im Bereich der Öffnungen 17 bis 22, vorzugsweise etwa in der Mitte derselben angeordnet ist. Die Elektrode 31 kann wie dargestellt einen Kreisquerschnitt oder auch einen Polygonalquerschnitt aufweisen.

Von der Elektrode 31 kann sich ein elektrischer Leiter 32 bis zu dem proximalen Ende 14 des Instruments 11 erstrecken und dort an das Gerät 29 angeschlossen sein. Das Gerät 29 kann eine elektrische Quelle, beispielsweise in Gestalt eines Hochspannungs-Hochfrequenz-Generators 33 enthalten, über den die Elektrode 31 mit einer elektrischen Spannung speisbar ist. Die hochfrequente Spannung hat einen derartigen Wert, dass sie zur Ionisation des über das Lumen 15 herangeführten Gasstroms an der Elektrode 31 ausreicht, um ein Plasma zu bilden. Die Spannung beträgt üblicherweise mehrere 100 Volt bei einer Frequenz oberhalb 100 kHz, vorzugsweise mehrere 100 kHz, weiter vorzugweise jedoch unter 5 MHz.

Die Besonderheit der Erfindung liegt in der Ausbildung des Kopfs 16, der zumindest vorzugsweise einen sich zu der distalen Spitze 34 hin verjüngenden Querschnitt aufweist. Die Öffnungen 17 bis 22 und somit auch die Stege 23 - 28 können ganz oder teilweise in dem sich verjüngenden Bereich des Kopfs 16 angeordnet sein, wobei der sich verjüngende Bereich in Figur 2 etwa bei der Schnittlinie III-III beginnt. In dem sich verjüngenden Bereich nimmt der Durchmesser des Kopfs 16 kontinuierlich bis zu der distalen Spitze 34 hin ab, wo der Kopf 16 in einer Rundung vorzugsweise stumpf endet.

Die einzelnen Öffnungen 17 bis 22 sind voneinander durch die Stege 23 bis 28 getrennt, die gegenüber einer Längsrichtung L zumindest in Umfangsrichtung U geneigt angeordnet sind. Die Längsrichtung L verläuft längs durch das Lumen 15 und ist in Figur 2 durch eine Längsmittelachse 35 angegeben, die eine Symmetrieachse für eine Drehsymmetrie des Kopfs 16 bildet. Die Drehsymmetrie ist in dem Ausführungsbeispiel nach Figur 2 und 3 dreifach, d.h. bei einer Drehung des Kopfs um 120° um die Längsmittelachse 35 ist der gedrehte Kopf 16 deckungsgleich mit dem nicht gedrehten Kopf 16.

Die zwischen den Stegen 23 bis 28 begrenzten Öffnungen 17 bis 22 sind radial zu der Längsmittelachse 35 orientiert. In Figur 2 ist die Öffnungsrichtung des Fensters 17 durch einen gestrichelten Pfeil 36 markiert. Die Öffnungen 17 bis 22 öffnen sich demnach quer zu der Längsmittelachse 35 und somit auch quer zur der Längsrichtung L in radialer Richtung R oder, wie durch den Pfeil 36 angedeutet, auch gegen die Radialrichtung R etwas geneigt. Die Radialrichtung R steht senkrecht auf der Längsrichtung L. Zur Bestimmung der Öffnungsrichtung kann der Kopf 16 zunächst ohne Fenster als sich zu der distalen Spitze 34 hin verjüngender Rotationskörper gedacht werden. Die Verjüngung kann, wie Figur 2 veranschaulicht, zu der distalen Spitze 34 hin zunehmen, sodass sich der Längsschnitt durch den Kopf 16 zum Beispiel an eine Parabelform oder eine Halbellipse annähert. Wird auf einem solchen noch öffnungslos gedachten Kopf 16 an der für die jeweilige Öffnung 17 bis 22 vorgesehenen Stelle eine Öffnung aufgezeichnet und im Flächenschwerpunkt derselben der Normalenvektor angetragen, bezeichnet dieser die Richtung des Pfeils 36.

Die Stege 23 bis 28 weisen einen sich in positiver Radialrichtung +R (radial nach außen) verjüngenden Querschnitt auf. Dieser Querschnitt ist in Figur 2 dünndick-schraffiert veranschaulicht. Vorzugsweise ist der Querschnitt dreieckig, wie aus Figur 3 ersichtlich und in Figur 4 am Beispiel des Stegs 28 veranschaulicht. Der Steg 28 ist in der gleichen Schnittebene III-III geschnitten wie in Figur 2 eingezeichnet und in Figur 3 dargestellt. Durch die Dickenabnahme der Stege 23 bis 28 radial nach außen nimmt der Fensterquerschnitt der Öffnungen 17 bis 22 radial nach außen hin zu.

Der in Figur 4 stellvertretend für alle Stege 23 - 28 veranschaulichte Querschnitt des Stegs 28 weist eine der Längsmittelachse 35 zugewandte Seitenfläche 37 sowie zwei weitere Seitenflächen 38, 39 auf, die die Öffnungen 22 und 17 seitlich begrenzen. Die Querschnitte der Stege 23 bis 28 sind entlang jedes Stegs 23 bis 28 vorzugsweise im Wesentlichen unverändert. Die ebenen oder auch konvex gerundet ausgebildeten Seitenflächen 38, 39 (Figur 3) des Stegs 23 schließen miteinander einen spitzen Winkel ein, der sich zu der Längsmittelachse 35 hin öffnet. Damit weist der Steg 28 mit seiner innen liegenden Seitenfläche 37 auf die Elektrode 31. Mit seiner vorzugsweise gerundeten Ecke 40 weist der Steg 28 von der Längsmittelachse 35 gesehen radial nach außen, d.h. von der Längsmittelachse 35 weg. Die übrigen Ecken 41, 42 sind vorzugsweise ebenfalls gerundet. Die Seitenflächen 38, 39 können eben oder vorzugsweise etwas konvex gerundet sein. Die Seitenfläche 37 kann eben oder, wie in Figur 4 dargestellt, konkav, bedarfsweise aber auch konvex gerundet sein.

Figur 5 veranschaulicht eine abgewandelte Ausführungsform der erfindungsgemäßen Plasmasonde, bei der der Stegquerschnitt anders als in Figur 4 nicht dreieckig, sondern trapezförmig ausgebildet ist. Anstelle der gerundeten Ecke 40 ist nun eine gerade oder gerundete Kante 40' vorgesehen. Ansonsten gelten die vorigen Ausführungen zu den Seitenflächen 38, 39 sowie zu der Innenfläche 37 entsprechend.

Allen bislang vorhandenen Ausführungsformen ist gemein, dass die Elektrode 31 einen Durchmesser D aufweist, siehe Figur 3, der vorzugsweise größer ist als die Stegbreite BS, die an der breitesten Stelle jedes Stegs 23 bis 28 jeweils in Umfangsrichtung U zu messen ist. Dadurch wird eine Minimierung der Abschattung der Elektrode 31 nach au-ßen erreicht. Werden in Figur 3 gestrichelt dargestellte Geraden G1, G2 so eingetragen, dass diese einerseits die Elektrode 31 an einander gegenüberliegenden Seiten und andererseits den Steg 23 an seinen Seitenflächen 38, 39 berühren, schneiden sich diese Geraden G1, G2 in einem Punkt P1 außerhalb des Kopfs 16, wobei der betreffende Steg 23 zwischen dem Punkt P1 und der Elektrode 31 liegt. Werden Geraden G3 und G4 an die das Fenster 17 begrenzenden Seitenflächen der Stege 23, 28 gelegt, schneiden sich diese Geraden G3, G4 innerhalb des Kopfs 16, vorzugsweise in dem Zwischenraum zwischen der Elektrode 31 und den Stegen 23, 28. Weist die Elektrode 31 anstelle eine Kreisquerschnitts einen Polygonalquerschnitt auf, tritt an die Stelle des Durchmessers D die Diagonale des Polygonalquerschnitts. Die Elektrode 31 kann als Einzelelektrode mit rundem oder polygonalem Querschnitt ausgebildet sein. Die Elektrode 31 kann auch durch ein Bündel von einzelnen elektrischen Leitern gebildet sein, die in Abständen zueinander oder aneinander anliegend angeordnet sind. Der Durchmesser D kennzeichnet dann den Außendurchmesser des Querschnitts des Bündels von Leitern.

Die Breite BS des Stegs 23 ist vorzugsweise geringer als die Breite BE zumindest einer benachbarten Öffnung, in Figur 3 der Öffnung 18, gleichfalls gemessen in Umfangsrichtung U. Dies gilt zumindest an einer Axialposition, wobei die Breite BS und die Breite BE an der gleichen Axialposition gemessen werden. Auch dies sichert die Kontinuität des Plasmastroms, der somit durch den Steg 23 wie auch durch die anderen Stege 24 bis 28 nicht unterbrochen wird. Die genannte Bedingung für die Breiten BS und BE (BE > BS) gilt jeweils mindestens dort, wo die Öffnungen ihre größte Ausdehnung in Umfangsrichtung U haben.

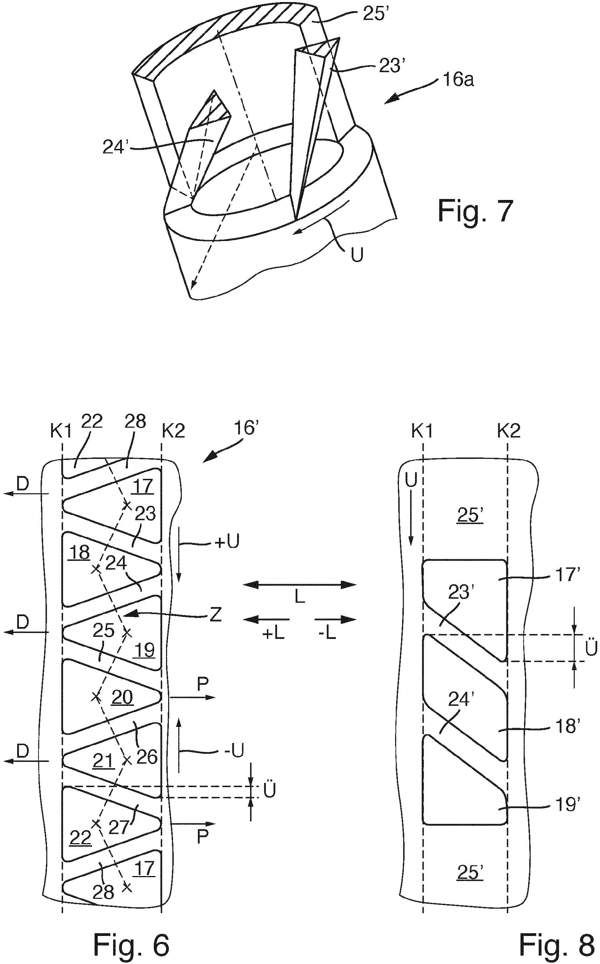

Figur 6 veranschaulicht eine Abwicklung 16' des Kopfs 16, wobei sich die Enden der Abwicklung überlappen. Die sechs Fenster 17 bis 22 sind bspw. dreieckig. Benachbarte Stege 23 bis 28 sind jeweils paarweise gegensinnig in positiver Umfangsrichtung +U und negativer Umfangsrichtung -U geneigt. Dabei stimmen die Beträge der Neigungswinkel, den die einzelnen Stege 23 bis 28 mit der Umfangsrichtung U einschließen, miteinander überein. Mit anderen Worten, der Neigungswinkel jedes Stegs 23 bis 28 wechselt im Verlauf der Umfangsrichtung U von Steg zu Steg jeweils sein Vorzeichen. Dadurch erhalten die Öffnungen 17 bis 22 jeweils eine dreieckige Grundform mit mehr oder weniger stark gerundeten Ecken, wobei die Basen der durch die Öffnungen 17 bis 22 markierten Dreiecke auf zwei zueinander parallelen Kreisen K1, K2 gleichen oder leicht unterschiedlichen Durchmessers liegen. Die Mittelpunkte der Kreise K1 und K2 liegen auf der Längsmittelachse 35. Die Spitzen der durch die Öffnungen 17 bis 22 markierten Dreiecke weisen abwechselnd in distaler Richtung D (d.h. positive Längsrichtung +L) oder proximaler Richtung P (d.h. negativer Längsrichtung -L). Die im Wesentlichen dreieckigen Öffnungen 17 bis 22 weisen Flächenschwerpunkte auf, die wie in Figur 5 angedeutet auf einer Zick-Zack-Linie Z liegen können.

Die Neigungen bzw. Neigungswinkel der Stege 23 bis 28 sind so bemessen, dass sich die Öffnungen 17 bis 22 in Umfangsrichtung U überlappen. Die Umfangsrichtung ist durch den Verlauf der hier durch die Abwicklung als Geraden veranschaulichten Kreise K1, K2 gekennzeichnet. In Figur 6 ist die Überlappung am Beispiel des Stegs 27 und den Öffnungen 21, 22 veranschaulicht. Die in positiver Umfangsrichtung +U vorn liegende Ecke der Öffnung 21 und die in positiver Umfangsrichtung +U hinten liegende Öffnung der Ecke 22 überlappen einander um den Überlappungsbetrag Ü. Dieser ist mindestens Null und vorzugsweise größer als Null. Mit anderen Worten, wird die Öffnung 21 gedanklich in distaler Richtung D und/oder die Öffnung 22 gedanklich in proximaler Richtung P verschoben, berühren oder überlagern die Öffnungen 21, 22 einander.

Bei dem Ausführungsbeispiel nach Figur 1 bis 6 sind die Stege 23 bis 28 paarweise gegensinnig in und gegen die Umfangsrichtung (d.h. in +U und -U) geneigt. Es ist jedoch auch möglich, Stege 23', 24` gleichsinnig zu neigen, wie anhand eines Ausführungsbeispiels nach den Figuren 7 und 8 veranschaulicht ist. Während es sich bei dem vorbeschriebenen Instrument 11 um ein Instrument mit 360° Rundumwirksamkeit handelt, ist das Instrument 11` nach Figur 7 zur Gewebebehandlung lediglich in einem eingeschränkten Umfangsbereich vorgesehen. Der Kopf 16a weist einen breiteren Abschnitt 25` auf, der mit oder ohne Neigung in Umfangsrichtung ausgebildet ist und als gebogene Wand gedacht werden kann. Es ergeben sich drei Öffnungen 17', 18', 19`, die trapezförmig und rautenförmig ausgebildet sein können. Es überlappen sich wiederum die trapezförmige Öffnung 17' und die rautenförmige Öffnung 18' in Umfangsrichtung U. Gleiches gilt für die Öffnung 18' und die trapezförmige Öffnung 19`. Im Übrigen gilt die vorige Beschreibung unter Zugrundelegung der bereits eingeführten Bezugszeichen entsprechend.

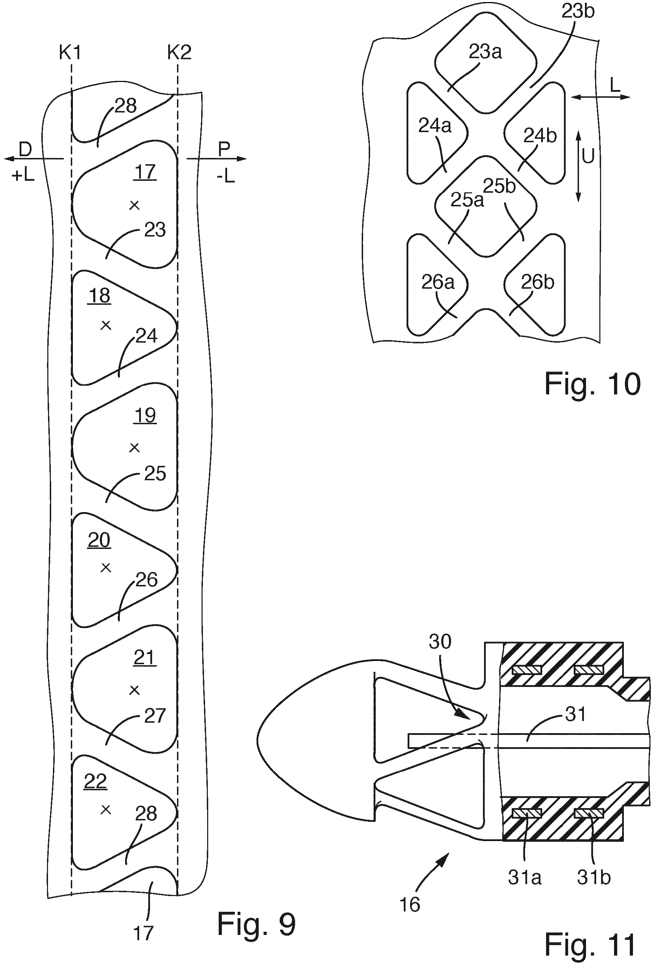

Weitere Abwandlungen sind möglich. Figur 9 zeigt dazu eine Abwicklung eines modifizierten Kopfs 16, für den die Beschreibung der Ausführungsform nach den Figuren 1 bis 6 mit Ausnahme der nachfolgend genannten Besonderheiten vollständig entsprechend gilt:

Wenigstens einige der Öffnungen 17 bis 22 oder auch alle Öffnungen 17 bis 22 sind nicht dreieck- sondern trapezförmig ausgebildet. Dazu ist anstelle der jeweiligen in distaler Richtung D oder proximaler Richtung P weisenden Spitze jeder Öffnung 17 bis 22 eine starke Rundung oder auch eine kurze Kante vorgesehen, die über zwei Rundungen in den jeweiligen Steg 23 bis 28 übergeht.

Die bislang beschriebenen Ausführungsformen nutzen Öffnungen 17 bis 22, die zwischen den beiden gedachten Kreisen K1, K2 in einer einzigen Reihe angeordnet sind. Es sind jedoch auch mehrreihige Anordnungen möglich, wie Figur 10 veranschaulicht. Die Besonderheit dieser aus insgesamt drei Reihen gerundeter drei- oder viereckiger Öffnungen bestehenden Anordnung sind wiederum geneigte Stege 23a, 23b, 24a, 24b, 25a, 25b, usw., die in der oder gegen die Umfangsrichtung U geneigt und somit nichtparallel zu der Längsrichtung L angeordnet sind. Wiederum überlappen die einzelnen Öffnungen in Umfangsrichtung einander.

Eine weitere Abwandlung ist hinsichtlich der Ausbildung der Plasmaerzeugungseinrichtung 30 möglich. Bei den bislang beschriebenen Ausführungsformen ist als Plasmaerzeugungseinrichtung 30 lediglich eine blanke Elektrode 31 vorgesehen, um ein weitgehend thermisches Plasma zu erzeugen. Es handelt sich um monopolare Instrumente, bei denen von der Elektrode 31 ein elektrischer Strom durch das Plasma zu dem Gewebe und von diesem über eine Neutralelektrode N zu dem Generator 33 zurück fließt. Es können aber bei allen vorbeschriebenen Ausführungsformen alternativ auch ein oder mehrere elektrisch isoliert angeordnete Elektroden 31a, 31b zur Ausbildung einer Barriereentladung vorgesehen sein. Beispielsweise kann die Elektrode 31a und/oder die Elektrode 31b als schalen- oder ringförmige Elektrode in das Material des Kopfs 16 eingebettet oder auf andere Weise isoliert in diesem angeordnet sein. Die beiden Pole des Generators können mit den isolierten Elektroden 31a, 31b verbunden sein, um eine Barriereentladung zu erzeugen. In diesem Fall kann die Elektrode 31 entfallen. Alternativ können eine oder beide Elektroden 31a, 31b mit einem Pol des Generators und die mit einer Isolierschicht versehene oder blanke Elektrode 31 mit dem anderen Pol des Generators verbunden sein, um eine Barriereentladung zu bilden. Solche Anordnungen dienen insbesondere zur Erzeugung von nicht thermischem warmem oder kaltem Plasma.

Die verschiedenen hier kurz beschriebenen Plasmaerzeugungseinrichtungen 30 können mit jedem der vorstehend beschriebenen Köpfe nach Figur 1 bis 9 kombiniert werden.

Das im Zusammenhang mit Figur 3 beschriebene Grö-ßenverhältnis, wonach der Durchmesser D der Elektrode 31 zumindest so groß wie, vorzugsweise größer als, die Breite BS des Stegs 23 (und auch weiterer Stege) ist, ist auch bei Sonden mit seitlichen Fenstern gemäß der vorigen und nachstehenden Beschreibung vorteilhaft, bei denen sich aber Steg 23 (und auch weitere Stege) nicht nach außen verjüngen. Solche Stege können z.B. einen quadratischen oder rechteckigen Querschnitt mit geraden oder gerundeten Kanten und spitzen oder gerundeten Ecken aufweisen. Auch können die Stege einen Rundquerschnitt aufweisen.

Das insoweit beschriebene Instrument 11 arbeitet wie folgt:
In Betrieb wird das Instrument 11 von dem Gerät 29 mit Gas, beispielsweise Argon versorgt, so dass dieses durch das Lumen 15 hindurch und aus den Öffnungen 17 - 22 ausströmt. Außerdem wird die elektrische Quelle 33 aktiviert, so dass an der Plasmaerzeugungseinrichtung 30 eine elektrische Entladung zur Ionisierung des Gasstroms und zur Erzeugung von Plasma stattfindet. Insbesondere in der monopolaren Variante, bei der ein Pol der elektrischen Quelle 33 mit der vorzugsweise nackten Elektrode 31 und der andere Pol der Quelle 33 mit dem Patienten verbunden ist, bildet sich eine elektrische Entladung zwischen der Elektrode 31 und dem Kopf 16 nächstgelegenen biologischen Gewebe, so dass sich ein Plasmastrom PS durch die dazwischenliegende Öffnung, zum Beispiel die Öffnung 19 etabliert. Dieser Zustand ist in Figur 1 veranschaulicht.

Wird nun das Instrument 11 bewegt, kann es sein, dass die Entladung, das heißt der Plasmastrom PS, seinen Weg durch das benachbarte Fenster 18 oder 20 sucht. Der Übergang des Plasmastroms von dem Fenster 19 zu dem Fenster 18 oder 20 ist dabei kontinuierlich und glatt, weil die Fenster, wie am Beispiel aus Figur 5 ersichtlich, einander insbesondere bei Blickrichtung in Längsrichtung L in Umfangsrichtung überlappen. Die schräg gestellten und sich radial nach außen verjüngenden Stege 23 bis 28 sind der Entladung somit nicht im Weg, was die Sprunghaftigkeit des vom Behandler als Funken wahrgenommenen Plasmastrahls gegenüber Instrumenten mit in Längsrichtung orientierten Stegen wesentlich reduziert.

Wird als Plasmaerzeugungseinrichtung 30 eine Vorrichtung mit Barriereentladung wie gemäß Figur 10 veranschaulicht genutzt, kann erreicht werden, dass das Plasma in einem alle Öffnungen 17 bis 22 lückenlos einnehmenden ringförmigen Bereich entsteht, wobei Abschattungen durch die Stege 23 bis 28 wiederum minimiert sind.

Ein erfindungsgemäßes Instrument 11 zur Plasmabehandlung von biologischem Gewebe weist Plasmaaustrittsfenster in Gestalt von Öffnungen 17 - 22 mit einem zur Längsrichtung L schräg verlaufenden Rand auf, der durch eine Flanke 38, 39 eines Stegs gebildet sein kann. Die Stege 23 - 28 verjüngen sich in Radialrichtung +R und sind vorzugsweise schräg zu der Längsrichtung L geneigt. Die Stege 23 bis 28 tragen einen proximalen Abschnitt des Instrumentenkopfs 16 und verbinden diesen nahtlos einstückig mit dem proximalen Teil des Kopfs 16. Durch die Verjüngung der Stege nach außen wird deren Abschattungswirkung minimiert oder beseitigt.

### Bezugszeichen:

- 11: Instrument
- 12: Körper
- 13: distales Ende des Körpers
- 14: proximales Ende des Körpers
- 15: Lumen
- 16: Kopf
- 16': Abwicklung des Kopfs 16
- 17 - 22: Öffnungen
- 23 - 28: Stege
- 29: Gerät
- 30: Plasmaerzeugungseinrichtung
- 31: Elektrode
- 32: Leiter
- G: Gasquelle
- 33: elektrische Quelle
- 34: distale Spitze des Kopfs 16
- L: Längsrichtung, +L, -L
- R: Radialrichtung, +R, -R
- 35: Längsmittelachse
- 36: Pfeil der Öffnungsrichtung
- 37: Innere Seitenfläche des Stegs 28
- 38, 39: Seitenflächen an den Fensterflanken
- 40 - 42: Ecken des Stegquerschnitts
- 40': äußere Begrenzung des Stegquerschnitts
- K1, K2: Kreise
- U: Umfangsrichtung +U, -U
- Ü: Überlappungsbetrag
- D: distale Richtung
- P: proximale Richtung
- Z: Zickzacklinie
- PS: Plasmastrom
- N: Neutralelektrode

## Patentansprüche

1. Plasmainstrument (11) zur Einwirkung auf biologisches Gewebe, insbesondere zur Behandlung von menschlichen oder tierischen Patienten,
mit einem länglichen, als Schlauch oder Rohr ausgebildeten Körper (12), der ein distales Ende (13) und ein proximales Ende (14) sowie ein sich entlang einer Längsrichtung (L) von dem proximalen Ende (14) bis zu dem distalen Ende (13) erstreckendes Lumen (15) aufweist, das an eine Gasquelle (G) angeschlossen oder anschließbar ist,
mit einem an das distale Ende (13) angeschlossenen Kopf (16), der wenigstens zwei mit dem Lumen (15) verbundene Öffnungen (17, 18) aufweist, die voneinander durch einen Steg (23) getrennt sind, der einen sich in einer Radialrichtung (R) nach außen verjüngenden Querschnitt aufweist.

2. Plasmainstrument nach Anspruch 1, **dadurch gekennzeichnet, dass** der in dem Körper (12) und/oder dem Kopf (16) eine Elektrode (31) angeordnet ist, die über einen sich zu dem proximalen Ende (14) ersteckenden elektrischen Leiter (32) an einen elektrochirurgischen Generator (G) anschließbar ist.

3. Plasmainstrument nach Anspruch 2, **dadurch gekennzeichnet, dass** die Elektrode (31) einen Durchmesser (D) und der Steg (23) an seiner der Elektrode (31) zugewandten Seite eine Breite (BS) aufweist, die geringer ist, als der Durchmesser (D) der Elektrode (31) .

4. Plasmainstrument nach einem der vorstehenden Ansprüche 2 oder 3, **dadurch gekennzeichnet, dass** die Elektrode (31) einen Kreisquerschnitt aufweist.

5. Plasmainstrument nach einem der vorstehenden Ansprüche 2 bis 4, **dadurch gekennzeichnet, dass** der Steg (23) an seiner der Elektrode (31) zugewandten Seite (37) eben oder konvex oder konkav ausgebildet ist.

6. Plasmainstrument nach einem der vorstehenden Ansprüche, **dadurch gekennzeichnet, dass** der Querschnitt des Stegs (23) trapezförmig oder dreieckig ausgebildet ist.

7. Plasmainstrument nach Anspruch 6, **dadurch gekennzeichnet, dass** der Querschnitt des Stegs (23) gerundete Ecken (40, 41, 42) aufweist.

8. Plasmainstrument nach einem der vorstehenden Ansprüche, **dadurch gekennzeichnet, dass** der Steg (23) und die Öffnung (17) in einer sowohl zu der Längsrichtung (L) als auch zu der Radialrichtung (R) rechtwinklig orientierten Umfangsrichtung (U) jeweils eine Breite (BE, BS) aufweisen, wobei die Breite (BE) größer ist als die Breite (BS) des Stegs (23).

9. Plasmainstrument nach einem der vorstehenden Ansprüche, **dadurch gekennzeichnet, dass** der Kopf (16) aus einem temperaturbeständigen Material nahtlos einstückig ausgebildet ist.

10. Plasmainstrument nach einem der vorstehenden Ansprüche, **dadurch gekennzeichnet, dass** die in dem Kopf (16) ausgebildeten Öffnungen (17 - 22) eine sich um den gesamten Kopf (16) herum erstreckende Reihe bilden.

11. Plasmainstrument nach einem der vorstehenden Ansprüche, **dadurch gekennzeichnet, dass** der Steg (23) schräg zu der Längsrichtung (L) ausgerichtet ist.

12. Plasmainstrument nach Anspruch 10, **dadurch gekennzeichnet, dass** zwischen den Öffnungen (17 - 22) Stege (23 - 28) angeordnet sind, die in Umfangsrichtung (U) mit abwechselndem Richtungssinn (+U, -U) geneigt sind.

13. Plasmainstrument nach einem der vorstehenden Ansprüche, **dadurch gekennzeichnet, dass** die Öffnungen (17 - 22) sich in Längsrichtung (L) mit abwechselndem Richtungssinn (+L, -L) verjüngend ausgebildet sind.

14. Plasmainstrument nach einem der vorstehenden Ansprüche, **dadurch gekennzeichnet, dass** die Öffnungen (17 - 22) in Umfangsrichtung (U) und in Längsrichtung (L) einander überlappend ausgebildet sind.

15. Plasmainstrument nach einem der vorstehenden Ansprüche, **dadurch gekennzeichnet, dass** die Öffnungen (17 - 22) trapez- oder dreieckförmig mit gerundeten Ecken ausgebildet sind.
